# EUROPEAN PATENT APPLICATION

(11) **EP 3 730 133 A1**
(43) Date of publication of application: **28.10.2020**
(21) Application number: 18892327.0
(22) Date of filing: 18.05.2018
(51) Int. Cl.: A61K 31/198, A61K 9/08

(54) **FUDOSTEINE SOLUTION PREPARATION FOR AEROSOL INHALATION, AND PREPARATION METHOD THEREFOR**

(30) Priority: 19.12.2017 CN 201711373995
(71) Applicant: Beijing Increase Innovation Drug Research Co., Ltd, Beijing 102200 (CN)
(72) Inventor: ZHANG, Baoxian, Beijing 102200 (CN); HU, Jie, Beijing 102200 (CN)
(74) Representative: Seemann & Partner Patentanwälte mbB
(86) International application number: PCT/CN2018/087524
(87) International publication number: WO 2019/119720

(57) **Abstract**

Disclosed are a fudosteine solution preparation for aerosol inhalation, and a preparation method therefor. The fudosteine solution preparation for aerosol inhalation comprises fudosteine, salts thereof and/or hydrates thereof; a metal complexing agent; and water for injection. The method for preparing the fudosteine solution preparation for aerosol inhalation includes the following steps: adding water for injection to a liquid formulation device and filling in nitrogen for protection, keeping the nitrogen in a liquid formulation tank at a positive pressure, and determining a residual oxygen content to be less than 2 mg/L; weighing the metal complexing agent, and stirring until the same dissolves; adding fudosteine, salts thereof and/or hydrates thereof, and stirring until the same dissolves; adding a pH adjusting agent, and determining the residual oxygen content to be less than 2 mg/L; supplementing with water for injection to a full amount, and stirring until evenly mixed; and carrying out fine filtration under sterile conditions, encapsulating and filling in nitrogen.

## Description

### CROSS-REFERENCE TO EARLIER FILED APPLICATIONS

This application claims the benefit of and is a continuation of PCT/CN2018/087524, filed on May 18, 2018, which claims the benefit of CN 201711373995.6, filed on December 19, 2017, the entire disclosures of which are hereby incorporated by reference.

### TECHNICAL FIELD

The present disclosure belongs to the technical field of pharmaceutical preparation, and specifically relates to a fudosteine solution preparation for aerosol inhalation and a preparation method therefor.

### BACKGROUND

Due to factors such as the dense population, the large number of smoking people and environmental pollution, the morbidity and mortality of respiratory system diseases are high in recent years. According to the data of American Statistical Yearbook, among all categories of causes of death, the ranking of the death caused by respiratory tract-related diseases (excluding tumors) has increased from the 10th in 1970 to the 4th (chronic obstructive pulmonary disease) and the 8th (pneumonia, influenza and upper respiratory tract infection) in 1991, and the morbidity of respiratory system diseases in China ranks first among various diseases in any age group. This problem has increasingly aroused people's attention, and therefore the development of drugs for treating respiratory system diseases (simply referred to as respiratory drugs) has also become an important research and development field of drug research.

Cough and expectoration are common symptoms of respiratory system diseases in clinic. Excessively frequent and severe cough not only increases pain of patients, influences rest and sleep, increases consumption of physical strength, but also even promotes the progression of diseases, and causes other complications, such as pneumonia, chronic pharyngitis, chronic bronchitis, bronchiectasis, pulmonary abscess, cavitary pulmonary tuberculosis, and the like. In such cases, in addition to the symptomatic treatment aiming at the respiratory system diseases, antitussive and expectorant drugs are also needed to be properly applied to relieve cough. The application of antitussive drug(s) is only to relieve symptoms, and fundamentally, the treatment should aim at the etiology of cough. As most of the cough is caused by inflammatory mediators, for example, the mediators excessively released in diseases such as tracheitis, asthma, pneumonia, lung tumors, and the like, expectorants are further needed to be used according to the state of a disease.

Fudosteine is a novel expectorant belonging to cysteine derivatives. Fudosteine was first launched in Japan on December 17, 2001. Its basic pharmacological effects comprise inhibiting goblet cell hyperplasia, breaking the disulfide bond of mucin in bronchial secretions, and regulating the normal state of mucus and mucous membrane in the respiratory tract. Fudosteine is an expectorant with high efficiency and low toxicity and is expected to become a new replacement product of the similar drugs such as bromhexine, acetylcysteine, carboxymethylcysteine and the like. Fudosteine is a relatively stable compound. When it exists alone in the air, it is quite stable even under high humidity without the occurrence of discoloration. However, when fudosteine is used together with fillers commonly used in a solid preparation, such as various saccharides, celluloses, and sugar alcohols, discoloration will occur, which not only affects the appearance of the sample but also causes a decrease in content sometimes.

Currently, only tablets, granules and capsules have been launched in China, most of which are oral preparations with poor fluidity, easy to absorb moisture and thus affecting the long-term stability of the preparations, and may cause certain damage to gastrointestinal mucosa during administration.

### SUMMARY

### Technical problem

In view of the above, the technical problem to be solved by the present disclosure is to provide a fudosteine solution preparation for aerosol inhalation by the present disclosure, which is used for treating diseases such as sputum thickening, difficulty in expectoration and phlegm obstruction in trachea caused by chronic bronchitis, bronchial asthma and the like. The fudosteine solution preparation for aerosol inhalation of the present disclosure is directly inhaled from mouth and nose, thereby avoiding the first-pass effect in liver and the damage and degradation in gastrointestinal tract, and greatly reducing the metabolic process of the drug by liver and kidney and significantly reducing the damage to the organ(s) of a patient. The fudosteine solution preparation for aerosol inhalation of the present disclosure makes up for the blank in the current domestic market. The present disclosure provides a novel, safe and effective drug preparation and method of administration of fudosteine.

### Solution to the problems

The present disclosure provides a fudosteine solution preparation for aerosol inhalation comprising: fudosteine, a salt thereof and/or a hydrate thereof; a metal complexing agent; a pH adjusting agent; and water for injection.

In a preferred embodiment, the preparation also comprises one or more pharmaceutical excipients suitable for pulmonary administration, wherein the pharmaceutical excipient comprises an antioxidant and a surfactant.

In a preferred embodiment, a single dose of the preparation comprises: 20 to 120 mg of fudosteine, a salt thereof and/or a hydrate thereof, calculated as free fudosteine; 0.1 to 5 mg of the metal complexing agent; a suitable amount of the pH adjusting agent; and water for injection.

In a preferred embodiment, a single dose of the preparation comprises: 50 to 100 mg of fudosteine, a salt thereof and/or a hydrate thereof, calculated as free fudosteine; 0.5 to 2 mg of the metal complexing agent; a suitable amount of the pH adjusting agent; and water for injection.

In a preferred embodiment, the pH adjusting agent is hydrochloric acid or sulfuric acid.

In a preferred embodiment, the preparation has a pH value of 3 to 9, preferably 3.5 to 5.5, and more preferably 3.5 to 5.0.

In a preferred embodiment, the metal complexing agent comprises at least one selected from the group consisting of edetic acid, disodium edetate, and calcium disodium edetate.

In a preferred embodiment, the preparation is used for treating the following conditions: sputum thickening, difficulty in expectoration and phlegm obstruction in trachea caused by chronic bronchitis and bronchial asthma.

The present disclosure also provides a preparation method of said preparation comprising the following steps:
(1) adding 50% to 80% of the total amount of water for injection into a liquid formulation device, controlling a water temperature at 25 ± 10°C, filling nitrogen into the water for injection for protection until a liquid formulation is finished, keeping a positive nitrogen pressure in a liquid formulation tank, and carrying out the next operation after a residual oxygen content is measured to be less than 2 mg/L;
(2) weighing the metal complexing agent, slowly adding the metal complexing agent into the above water for injection, and stirring until the metal complexing agent is completely dissolved;
(3) slowly adding fudosteine, a salt thereof and/or a hydrate thereof, and stirring until complete dissolution;
(4) adding the pH adjusting agent to adjust pH, simultaneously measuring the residual oxygen content, and carrying out the next operation after the residual oxygen content is less than 2 mg/L;
(5) further adding the rest of the total amount of water for injection and stirring to mix uniformly; and
(6) using a 0.45-µm filter membrane for a primary filtration and a 0.22-µm filter membrane for a fine filtration, both filtrations being sterile filtrations, bottling and encapsulating the resultant in an ampoule and filling the ampoule with nitrogen gas, wherein a filling volume is 5 ml.

### Advantageous effects

Compared with the prior art, the advantageous effects of the present disclosure are as follows:
(1) Compared with other dosage forms, the drug directly reaches the target organ(s), thereby avoiding the first-pass effect in liver and the damage and degradation in gastrointestinal tract and reducing the damage to the organs of the whole body, especially the damage to liver and kidney, and is safer to use.
(2) Since the fudosteine solution preparation for aerosol inhalation of the present disclosure is an inhalation-type preparation, it has a faster effect than an oral dosage form for respiratory system diseases, and avoids the defect that the drug needs to be absorbed through gastrointestinal tract first and then exerts systemic effect along with blood circulation and thus the onset of action is slow.
(3) The stability of fudosteine is improved, various detection indexes do not significantly change after the preparation is left to stand for a long period of time, and the product quality is ensured as qualified within the validity period.
(4) The types of excipients added are few, the safety of medication is high, the production process is simple, the cost is low, and the industrial scale production can be realized.

### DETAILED DESCRIPTION

Various exemplary examples, features and aspects of the present disclosure are described in detail below.

In addition, numerous specific details are set forth in the specific embodiments below in order to better illustrate the present disclosure. It should be understood by those skilled in the art that the present disclosure may also be implemented without some of these specific details. The following examples are merely for illustrating the present disclosure and should not be construed as limiting the scope of the present disclosure. Any equivalent replacement in the art made in accordance with the content of the present disclosure is within the protection scope of the present disclosure.

The fudosteine solution preparation for aerosol inhalation of the present disclosure comprises: fudosteine, a salt thereof and/or a hydrate thereof; a metal complexing agent; a pH adjusting agent; and water for injection.

By using the fudosteine solution preparation for aerosol inhalation of the present disclosure, conditions such as sputum thickening, difficulty in expectoration and phlegm obstruction in trachea caused by chronic bronchitis, bronchial asthma and the like are able to be treated. In addition, the fudosteine solution preparation for aerosol inhalation of the present disclosure is directly inhaled from mouth and nose, thereby avoiding the first-pass effect in liver and the damage and degradation in gastrointestinal tract, greatly reducing the metabolic process of the drug by liver and kidney and significantly reducing the damage to the organ(s) of a patient. Also, it has a faster effect than an oral dosage form for respiratory system diseases, and avoids the defect that the drug needs to be absorbed through gastrointestinal tract first and then exerts systemic effect along with blood circulation and thus the onset of action is slow.

The fudosteine solution preparation for aerosol inhalation of the present disclosure adopts the single-dose packaging for the drug. A single dose refers to the dose of the pharmaceutically active ingredient administered in a single inhalation. In the present specification, a single dose represents 5 mL.

The fudosteine solution preparation for aerosol inhalation provided by the present disclosure is a single-dose preparation. The preparation is convenient to use, does not need to be diluted and formulated, may greatly reduce microbial pollution and waste in the using process, and adopts a dose for single administration so as to avoid the defect caused by a multi-dose large-package solution, i.e., repeated measuring and repeated dilution and formulation are easy to cause the breeding of microbes.

The present disclosure provides a novel preparation having accurate drug dosage, superior and stable drug quality, and safe and simple clinical application which are lack in the prior art, and a preparation method thereof.

Each ingredient constituting the fudosteine solution preparation for aerosol inhalation of the present disclosure is described below.

### [Fudosteine, a salt thereof and/or a hydrate thereof]

In a single dose of the fudosteine solution preparation for aerosol inhalation of the present embodiment, calculated as free fudosteine, preferably, 20 to 120 mg of fudosteine, a salt thereof and/or a hydrate thereof is contained, and more preferably, 50 to 100 mg of fudosteine, a salt thereof and/or a hydrate thereof is contained.

When the content of fudosteine, a salt thereof and/or a hydrate thereof in a single dose of the fudosteine solution preparation for aerosol inhalation of the present disclosure is within the above range, the viscosity of sputum is able to be reduced, and the conditions such as sputum thickening, difficulty in expectoration and phlegm obstruction in trachea caused by chronic bronchitis, bronchial asthma and the like are favorably treated.

### [Metal complexing agent]

The metal complexing agent contained in the fudosteine solution preparation for aerosol inhalation of the present disclosure has complexing ability and is capable of improving the stability of the fudosteine solution preparation for aerosol inhalation.

As examples of the metal complexing agent, for example, edetic acid, edetate such as disodium edetate and calcium disodium edetate, and the like may be exemplified. Among these, edetate is preferred, and disodium edetate is more preferred. These metal complexing agents, especially edetate, have relatively strong ability to complex with metal ion(s) and the complexed ions have a plurality of types, so that the influence of the metal ions introduced in the liquid formulation process on the quality of the drug solution may be avoided. These metal complexing agents may be used alone, or may be used as a mixture of two or more.

In a single dose of the fudosteine solution preparation for aerosol inhalation, preferably, 0.1 to 5 mg of a metal complexing agent is contained, and more preferably, 0.5 to 2 mg of a metal complexing agent is contained. By containing the metal complexing agent in the above range, it is able to facilitate the exertion of the efficacy of the fudosteine solution preparation for aerosol inhalation and improve the stability of fudosteine. Various detection indexes do not significantly change even after the preparation is left to stand for a long period of time, and the product quality is ensured as qualified in the validity period.

If the content of the metal complexing agent is less than 0.1 mg, the stability of fudosteine becomes poor, various detection indexes change significantly after the preparation is left to stand for a long period of time, and the product quality is reduced. If the content of the metal complexing agent is greater than 5 mg, the ingested metal complexing agent such as disodium edetate may form a water-soluble chelate with the calcium ions in a human body and be excreted out of the body, and excessive intake of metal complexing agent may cause hypocalcemia or loss of bone calcium. Therefore, the content of the metal complexing agent such as disodium edetate in the product should be reduced as far as possible on the premise of ensuring effective antioxidation.

### [pH adjusting agent]

The fudosteine solution preparation for aerosol inhalation of the present disclosure contains a pH adjusting agent in order to adjust the pH value.

As a pH adjusting agent, it is not particularly limited as long as the above effects of the fudosteine solution preparation for aerosol inhalation are not impaired, for example, hydrochloric acid, sulfuric acid, lactic acid, malic acid, acetic acid, phosphoric acid, citric acid and the like may be exemplified. These pH adjusting agents may be used alone, or may also be used in combination. Among these, hydrochloric acid or sulfuric acid is more preferred.

The content of the pH adjusting agent may be determined appropriately according to the content of other ingredients, so that the pH value of the fudosteine solution preparation for aerosol inhalation is adjusted to a target value. By containing the pH adjusting agent, it is able to increase the stability of fudosteine solution.

The pH value of the fudosteine solution preparation for aerosol inhalation of the present disclosure is preferably 3 to 9, more preferably 3.5 to 5.5, and further preferably 3.5 to 5.0. By setting the pH value of the fudosteine solution preparation for aerosol inhalation within the above range, the solubility of fudosteine may be increased. If the pH value is less than 3, there is an adverse effect of excessive irritation to the human body after inhalation. On the other hand, if the pH value exceeds 9, there is a tendency that the stability of the solution becomes poor and the impurities increase, which is not preferred.

### [Pharmaceutical excipient]

The fudosteine solution preparation for aerosol inhalation of the present disclosure may contain one or more pharmaceutical excipients suitable for pulmonary administration if necessary.

The pharmaceutical excipient is not particularly limited, and examples thereof include an antioxidant, a surfactant, a flavoring agent, a stabilizer, and the like. Preferably, the pharmaceutical excipient includes an antioxidant and a surfactant. As an antioxidant, for example, sodium metabisulfite, sodium sulfite, sodium bisulfite, sodium thiosulfate, acetylcysteine and the like may be preferably exemplified. As a surfactant, for example, glycerin fatty acid ester, sorbitan fatty acid ester, polyoxyethylene fatty acid ester, polyethylene glycol fatty acid ester, sucrose fatty acid ester, and the like may be preferably exemplified.

### <Preparation method of the fudosteine solution preparation for aerosol inhalation>

In the present disclosure, the preparation steps of the fudosteine solution preparation for aerosol inhalation are not particularly limited, in principle, any method for preparing the preparation by mixing fudosteine, a salt thereof and/or a hydrate thereof, a metal complexing agent, a pH adjusting agent and water for injection is suitable for the present disclosure. The preparation method of the solution preparation of the present disclosure is preferably as follows:
(1) adding 50% to 80% of the total amount of water for injection into a liquid formulation device, controlling the water temperature at 25 ± 10°C, filling nitrogen into the water for injection for protection until a liquid formulation is finished, keeping a positive nitrogen pressure in the liquid formulation device, and carrying out the next operation after a residual oxygen content is measured to be less than 2 mg/L;
(2) weighing the metal complexing agent, slowly adding the metal complexing agent into the above water for injection, and stirring until the metal complexing agent is completely dissolved;
(3) slowly adding fudosteine, a salt thereof and/or a hydrate thereof, and stirring until complete dissolution;
(4) adding the pH adjusting agent to adjust pH, simultaneously measuring the residual oxygen content, and carrying out the next operation after the residual oxygen content is less than 2 mg/L;
(5) further adding the rest of the total amount of water for injection and stirring to mix uniformly; and
(6) using a 0.45-µm filter membrane for a primary filtration and a 0.22-µm filter membrane for a fine filtration, both filtrations being sterile filtrations, bottling and encapsulating the resultant in an ampoule and filling the ampoule with nitrogen gas, wherein a filling volume is 5 ml.

### EXAMPLES

The present disclosure is described in detail below with reference to Examples, but the protection scope of the present disclosure is not limited thereto.
Prescription 1: 1000 mL of fudosteine solution preparation for aerosol inhalation

| | |
|---|---|
| fudosteine | 4 g |
| disodium edetate | 20 mg |
| hydrochloric acid | suitable amount, pH adjusted to 3.0 |
| total volume reached by further adding water for injection | 1000 ml |

Prescription 2: 1000 mL of fudosteine solution preparation for aerosol inhalation

| | |
|---|---|
| fudosteine | 8g |
| disodium edetate | 100 mg |
| hydrochloric acid | suitable amount, pH adjusted to 3.5 |
| total volume reached by further adding water for injection | 1000 ml |

Prescription 3: 1000 mL of fudosteine solution preparation for aerosol inhalation

| | |
|---|---|
| fudosteine | 10g |
| disodium edetate | 200 mg |
| hydrochloric acid | suitable amount, pH adjusted to 4.0 |
| total volume reached by further adding water for injection | 1000 ml |

Prescription 4: 1000 mL of fudosteine solution preparation for aerosol inhalation

| | |
|---|---|
| fudosteine | 16 g |
| disodium edetate | 1 g |
| hydrochloric acid | suitable amount, pH adjusted to 4.5 |
| total volume reached by further adding water for injection | 1000 ml |

Prescription 5: 1000 mL of fudosteine solution preparation for aerosol inhalation

| | |
|---|---|
| fudosteine | 20 g |
| disodium edetate | 250 mg |
| sulfuric acid | suitable amount, pH adjusted to 5.0 |
| total volume reached by further adding water for injection | 1000ml |

Prescription 6: 1000 mL of fudosteine solution preparation for aerosol inhalation

| | |
|---|---|
| fudosteine | 24 g |
| disodium edetate | 800 mg |
| sulfuric acid | suitable amount, pH adjusted to 5.5 |
| total volume reached by further adding water for injection | 1000 ml |

Prescription 7: 1000 mL of fudosteine solution preparation for aerosol inhalation

| | |
|---|---|
| fudosteine | 24 g |
| edetic acid | 600 mg |
| sulfuric acid | suitable amount, pH adjusted to 4.0 |
| total volume reached by further adding water for injection | 1000 ml |

Prescription 8: 1000 mL of fudosteine solution preparation for aerosol inhalation

| | |
|---|---|
| fudosteine | 20 g |
| disodium edetate | 250 mg |
| hydrochloric acid | suitable amount, pH adjusted to 5.0 |
| total volume reached by further adding water for injection | 1000 ml |

Prescription 9: 1000 mL of fudosteine solution preparation for aerosol inhalation

| | |
|---|---|
| fudosteine | 20 g |
| disodium edetate | 250 mg |
| acetic acid and sodium acetate | suitable amount, pH adjusted to 5.0 |
| total volume reached by further adding water for injection | 1000 ml |

Prescription 10: 1000 mL of fudosteine solution preparation for aerosol inhalation

| | |
|---|---|
| fudosteine | 20 g |
| disodium edetate | 250 mg |
| phosphoric acid and sodium dihydrogen phosphate | suitable amount, pH adjusted to 5.0 |
| total volume reached by further adding water for injection | 1000 ml |

### Example 1: A fudosteine solution preparation for aerosol inhalation was prepared according to the proportion of Prescription 1.

70% of the total amount of water for injection, which was 700 mL, was added into a liquid formulation device, the water temperature was controlled at 30°C, nitrogen was filled into the water for injection for protection until the liquid formulation was finished, a positive nitrogen pressure was kept in the liquid formulation device, and the next operation was carried out after the residual oxygen content was measured to be 1.6 mg/L.

Then, 20 mg of disodium edetate (manufactured by Chengdu Huayi Pharmaceutical Excipient Manufacturing Co., Ltd.) was weighed and slowly added into the above water for injection, and the mixture was stirred until disodium edetate was completely dissolved.

Next, 4 g of fudosteine (manufactured by Weihai Disu Pharmaceutical Co., Ltd.) was slowly added, and the mixture was stirred until fudosteine was completely dissolved.

Then, a suitable amount of hydrochloric acid was added to adjust pH to 3.0, the residual oxygen content was simultaneously measured until it was 1.5 mg/L, and the next operation was carried out.

Next, water for injection was further added to full amount (1000 mL) and the mixture was stirred for 5 minutes to mix uniformly.

Then, the resultant was filtered with a 0.45-µm filter membrane for primary filtration and filtered with a 0.22-µm filter membrane for fine filtration, both filtrations were sterile filtrations. The resultant was bottled and encapsulated in an ampoule and the ampoule was filled with nitrogen gas, and the filling volume was 5 mL.

### Examples 2 to 10

In Examples 2 to 10, a fudosteine solution preparation for aerosol inhalation was prepared in the same manner as in Example 1, except that Prescriptions 2 to 10 were used in place of Prescription 1.

In order to further illustrate the technical effects of the present disclosure, the following specific experimental examples were provided.

### Experimental example 1

The single-dose fudosteine solution preparations for aerosol inhalation obtained in Examples 5 and 8 to 10 (the fudosteine solution preparations for aerosol inhalation were prepared according to the formulas of Prescriptions 5 and 8 to 10) were left to stand for one year. The contents of fudosteine in the single-dose fudosteine solution preparations for aerosol inhalation obtained in Examples 5 and 8 to 10 were determined by the following method for determining the content of fudosteine after one year, and the changes in color and clarity of the preparations were observed by visual observation. The results were shown in Table 1.

### Determination of the content of fudosteine

(1) Octadecylsilane-bonded silica gel was used as a filler (4.6 × 150 mm, 5 µm), a 1‰ phosphoric acid solution containing 5 mmol/L sodium heptanesulfonate solution was used as the mobile phase, the detection wavelength was 210 nm, the column temperature was 50°C, and the flow rate was 1.3 mL/min.
(2) An appropriate amount of the above test sample was precisely measured, a solution containing 0.5 mg of fudosteine per 1 mL was prepared by quantitatively diluting the test sample with the mobile phase, and the solution was used as a sample solution to be tested; in addition, an appropriate amount of fudosteine reference substance was precisely weighed, the mobile phase was added to dissolve and quantitatively dilute the fudosteine reference substance to prepare a solution containing 0.5 mg of fudosteine per 1 mL as a reference solution. 10 µL of the sample solution to be tested and 10 µL of the reference solution were each precisely measured and respectively injected into a liquid chromatograph, and the chromatograms were recorded. The content of fudosteine was obtained by calculating based on the peak area according to the external standard method.

**Table 1**

| pH adjusting agent | Color and clarity | Content of fudosteine |
|---|---|---|
| acetic acid and sodium acetate (Example 9) | slightly yellow, clear | 89.2% |
| phosphoric acid and sodium dihydrogen phosphate (Example 10) | slightly yellow, clear | 92.4% |
| hydrochloric acid (Example 8) | colorless, clear | 95.1% |
| sulfuric acid (Example 5) | colorless, clear | 95.3% |

As could be seen from Table 1, in a case where hydrochloric acid or sulfuric acid was used as a pH adjusting agent, even after the fudosteine solution preparation for aerosol inhalation was left to stand for one year, the content of fudosteine was still relatively high, the stability of fudosteine was excellent, and the change in color and clarity of the preparation was small. On the other hand, in a case where acetic acid and sodium acetate were used as pH adjusting agents and in a case where phosphoric acid and sodium dihydrogen phosphate were used as pH adjusting agents, after the fudosteine solution preparation for aerosol inhalation was left to stand for one year, the content of fudosteine was reduced, and the change in color and clarity of the preparation was relatively large.

### Experimental example 2

### Antitussive experiment (Concentrated Ammonia Water Spraying Method)

The experimental mice were placed in a specially-made glass bell jar, a quantitative spraying was carried out with 25% concentrated ammonia water by using an ultrasonic nebulizer, and the experimental mice were immediately taken out after 5 s. The latency period from the moment when the mice received spraying to the moment when the mice developed a cough, and the number of coughs within 2 min were recorded. The mice that did not cough 2 min after spraying were excluded. The qualified mice were randomly divided into a model group, an oral administration group, an injection administration group, and aerosol inhalation administration groups (low-, medium-, and high-dose groups) 2 days later.

The sodium chloride injection was administered via aerosol inhalation for the model group. The preparations used for the aerosol inhalation administration groups (low-, medium-, and high-dose groups) were obtained from Example 1, Example 3, and Example 5, respectively, and the administration dosages were 20, 40, and 100 mg/kg in sequence. The preparation used for the oral administration group and the injection administration group was obtained from Example 5, and the administration dosage was 100 mg/kg. The preparation was administered once a day for 5 consecutive days. Quantitative spraying was carried out with 25% concentrated ammonia water 1 h after the last administration, and the mice were immediately taken out after 5 s. The latency period from the moment when the mice received spraying to the moment when the mice developed a cough, and the number of coughs within 2 min were recorded. The results were shown in detail in Table 2, in which the antitussive effects in the model group as well as the oral administration group, the injection administration group and the aerosol inhalation administration groups (low-, medium-, and high-dose groups) of the fudosteine preparation were recorded.

**Table 2**

| Group | Latency period of cough/s | Number of coughs within 2 min/times | Suppression rate of cough % |
|---|---|---|---|
| model group | 12 ± 6 | 17.6 ± 7.1 | - |
| oral administration group | 43 ± 30** | 8.3 ± 4.9** | 63.7 |
| injection administration group | 51 ± 28** | 7.6 ± 3.1** | 74.6 |
| aerosol administration low-dose group | 64 ± 33** | 5.9 ± 3.9** | 84.9 |
| aerosol administration medium-dose group | 69 ± 29** | 3.8 ± 4.2** | 88.9 |
| aerosol administration high-dose group | 71 ± 35** | 2.9 ± 3.1** | 90.1 |

| | | | |
|---|---|---|---|
| **P < 0.01, as compared with the model group. | | | |

The results indicated that, as compared with the model group, oral administration, injection administration and aerosol inhalation administration of the fudosteine preparation were capable of significantly reducing the latency periods of cough in animals and reducing the number of coughs within 2 min, and the difference was extremely significant (P < 0.01). Among these, the antitussive effects in the aerosol inhalation administration groups (low-, medium-, and high-dose groups) were better than that in the oral administration group and the injection administration group in the aspects of prolonging the latency period of cough and reducing the number of coughs within 2 min.

### Experimental example 3

### Inhibitory experiment of goblet cell hyperplasia in the airway of mouse

### 3.1 Establishment of asthma model

60 mice were randomly divided into 6 groups with 10 mice in each group in accordance with the random number table method, including a model group, an oral administration group, an injection administration group, and aerosol inhalation administration groups (low-, medium-, and high-dose groups). The sodium chloride injection was administered via aerosol inhalation for the model group. The preparations used for the aerosol inhalation administration groups (low-, medium-, and high-dose groups) were prepared and obtained from Example 1, Example 3, and Example 5, respectively, and the administration dosages were 12.25, 24.50, and 61.25 mg/kg in sequence. The preparation used for the oral administration group and the injection administration group was prepared and obtained from Example 5, and the administration dosage was 100 mg/kg.

The mice in each group were sensitized by intraperitoneal injection of 0.2 mL of sensitizing solution (10 µg of OVA + 20 µg of aluminum hydroxide) on Days 1 and 14, respectively. From Day 21, the mice were challenged by being placed in an aerosol inhalation chamber with a size of about 0.5 m × 0.5 m × 0.5 m, and the mice were administered with 5 mL of 2.5% OVA solution via aerosol inhalation by using an ultrasonic nebulizer for 30 min each time (3 times a week for 6 consecutive weeks). In the normal saline group, the administration was replaced with an intragastric administration of normal saline. In the other five groups, the mice were respectively administered via intragastric administration, injection and aerosol inhalation in an amount of 0.1 mL (the drug preparation might be administered after being diluted properly) 10 min before asthma was aroused by each nebulization.

### 3.2 Specimen collection

24 h after of the last aerosol inhalation, a mouse was anesthetized with 10 g/L of sodium pentobarbital (40 mg/kg) via intraperitoneal injection, and then the main bronchus of the right lung was ligated. A 1-mL syringe was inserted into the left main bronchus, 40 g/L of paraformaldehyde-phosphate buffer solution was injected into the left lung under a pressure of 20 cm H₂O (1 cm H₂O = 0.098 kPa), and the left lung was incised and soaked in 40 g/L of paraformaldehyde-phosphate buffer solution for 24 h.

### 3.3 Cell counting of airway epithelial goblet cells

After the lung tissues were fixed with 40 g/L of paraformaldehyde-phosphate buffer solution, the tissues were routinely dehydrated and embedded, and stained with periodic acid-Schiff (PAS) reagent. 5 pieces of lung tissue sections were randomly selected from each mouse, and 5 bronchi with a round cross section and a diameter of 100 µm were randomly selected from each section by single-blind method for observation. The area of the goblet cells stained purple-red in the bronchial epithelium of each section was measured by using the image analysis software image-pro plus 6.0 and was expressed as a percentage with respect to the total area of the cells in the bronchial epithelium in which said goblet cells were located, and the number of the goblet cells was calculated. The details were shown in Table 3, in which the counting results of the airway epithelial goblet cells in different groups were shown.

**Table 3**

| Group | Number of goblet cells | Percentage of the area of goblet cells to the total area of epithelial cells % |
|---|---|---|
| model group | 166.13 ± 17.32 | 78.36 ± 8.72 |
| oral administration group | 103.42 ± 15.46** | 50.14 ± 6.38 |
| injection administration group | 97.22 ± 13.84** | 47.69 ± 5.46 |
| aerosol administration low-dose group | 65.41 ± 8.28** | 38.52 ± 3.83 |
| aerosol administration medium-dose group | 53.04 ± 6.39** | 32.05 ± 3.72 |
| aerosol administration high-dose group | 46.43 ± 4.59** | 27.31 ± 2.98 |

| | | |
|---|---|---|
| **P < 0.01, as compared with the model group. | | |

As compared with the model group, oral administration, injection administration and aerosol inhalation administration of the fudosteine preparation were capable of significantly reducing the number of goblet cells, and the results had extremely significant difference (P < 0.01). Among these, the aerosol inhalation administration groups (low-, medium-, and high-dose groups) showed better effects in the inhibitory experiment of goblet cell hyperplasia than that in the oral administration group and the injection administration group.

The above are only specific embodiments of the present disclosure, but the protection scope of the present disclosure is not limited thereto, and any changes or substitutions that may be easily conceived by those skilled in the art within the technical scope revealed by the present disclosure should be encompassed within the protection scope of the present disclosure. Therefore, the protection scope of the present disclosure should be subject to the protection scope of the claims.

## Claims

1. A fudosteine solution preparation for aerosol inhalation comprising: fudosteine, a salt thereof and/or a hydrate thereof; a metal complexing agent; a pH adjusting agent; and water for injection.

2. The preparation according to claim 1, wherein the preparation also comprises one or more pharmaceutical excipients suitable for pulmonary administration and the pharmaceutical excipient comprises an antioxidant and a surfactant.

3. The preparation according to claim 1 or 2, wherein a single dose of the preparation comprises: 20 to 120 mg of fudosteine, a salt thereof and/or a hydrate thereof, calculated as free fudosteine; 0.1 to 5 mg of the metal complexing agent; a suitable amount of the pH adjusting agent; and water for injection.

4. The preparation according to any one of claims 1 to 3, wherein a single dose of the preparation comprises: 50 to 100 mg of fudosteine, a salt thereof and/or a hydrate thereof, calculated as free fudosteine; 0.5 to 2 mg of the metal complexing agent; a suitable amount of the pH adjusting agent; and water for injection.

5. The preparation according to any one of claims 1 to 4, wherein the pH adjusting agent is hydrochloric acid or sulfuric acid.

6. The preparation according to any one of claims 1 to 5, wherein the preparation has a pH value of 3 to 9, preferably 3.5 to 5.5, and more preferably 3.5 to 5.0.

7. The preparation according to any one of claims 1 to 6, wherein the metal complexing agent comprises at least one selected from the group consisting of edetic acid, disodium edetate, and calcium disodium edetate.

8. The preparation according to any one of claims 1 to 7, wherein the preparation is used for treating the following conditions: sputum thickening, difficulty in expectoration and phlegm obstruction in trachea caused by chronic bronchitis and bronchial asthma.

9. A preparation method of the preparation according to any one of claims 1 to 8 comprising the following steps:
(1) adding 50% to 80% of the total amount of water for injection into a liquid formulation device, controlling a water temperature at 25 ± 10°C, filling nitrogen into the water for injection for protection until a liquid formulation is finished, keeping a positive nitrogen pressure in a liquid formulation tank, and carrying out the next operation after a residual oxygen content is measured to be less than 2 mg/L;
(2) weighing the metal complexing agent, slowly adding the metal complexing agent into the above water for injection, and stirring until the metal complexing agent is completely dissolved;
(3) slowly adding fudosteine, a salt thereof and/or a hydrate thereof, and stirring until complete dissolution;
(4) adding the pH adjusting agent to adjust pH, simultaneously measuring the residual oxygen content, and carrying out the next operation after the residual oxygen content is less than 2 mg/L;
(5) further adding the rest of the total amount of water for injection and stirring to mix uniformly; and
(6) using a 0.45-µm filter membrane for a primary filtration and a 0.22-µm filter membrane for a fine filtration, both filtrations being sterile filtrations, bottling and encapsulating the resultant in an ampoule and filling the ampoule with nitrogen gas, wherein a filling volume is 5 ml.
